# EUROPEAN PATENT APPLICATION

(11) **EP 1 818 063 A1**
(43) Date of publication of application: **15.08.2007**
(21) Application number: 05785934.0
(22) Date of filing: 20.09.2005
(51) Int. Cl.: A61K 45/00, A61K 31/12, A61K 31/196, A61P 9/00, A61P 9/10, A61P 25/08, A61P 25/28, G01N 33/15

(54) **MEDICINE FOR TREATMENT OF DISEASE CAUSED BY HYPERSTIMULATORY CYTOTOXICITY AND THERAPEUTIC METHOD**

(30) Priority: 02.12.2004 JP 2004350287
(71) Applicant: Japan Science and Technology Agency, Kawaguchi-shi, Saitama 332-0012 (JP); National Institutes of Natural Sciences, Tokyo (JP)
(72) Inventor: OKADA, Yasunobu, Aichi 444-0871 (JP); INOUE, Hana, Aichi 444-0860 (JP)
(74) Representative: Bentz, Jean-Paul
(86) International application number: PCT/JP2005/017317
(87) International publication number: WO 2006/059423

(57) **Abstract**

A medicine for treatment of diseases (for example, ischemic cerebrovascular disease) caused by hyperstimulatory cytotoxicity through protection or relief against hyperstimulatory cytotoxicity (for example, neurocytotoxicity); and a relevant therapeutic method. Further, there are provided a method of assessing any hyperstimulatory cytotoxicity and a method of screening a factor capable of inhibiting the cytotoxicity.

## Description

### TECHNICAL FIELD

The present invention relates to a medicine for treatment of diseases (for example, ischemic cerebrovascular disease) caused by excitotoxicity through protection or relief against excitotoxicity (for example, neuronal injury); and a relevant therapeutic method. Further, there are provided a method of assessing any excitotoxicity and a method of screening a factor inhibiting the cell injury.

### BACKGROUND ART

Brain tissue swelling, also known as brain edema, is an ischemic cerebrovascular disease whose probable cause is considered to be neurons' hyperexcitation induced by an excess glutamate release from neurons or glial cells. The neuronal swelling causing brain edema is excitotoxic and observed in brain ischemia, seizure, or other types of brain injuries. Prolonged neuronal swelling exerts pressure on surrounding cells and/or entire brain tissues, increasing intracranial pressure. This leads to very serious injuries, for example, such as brain herniation (see Non-Patent Publication 1 and Non-Patent Publication 2).

Alleviating brain edema can reduce damaged areas. In clinical settings, brain edema is commonly relieved by application of hypertonic solution. However, the method using hypertonic solution is associated with various side effects, such as dehydration, electrolyte abnormality, congestive heart failure, and serious kidney damage. Accordingly, there has been demand for a brain-edema relieving method that does not cause serious side effects.

Neuronal swelling is caused by large influx of sodium (Na⁺) via glutamate receptor channels, and influx of electrically obliged chloride (Cl-). Prolonged neuronal swelling caused by hyperexcitation leads cytotoxicity, i.e., irreversible cell death (necrosis) by cell rapture, in addition to injuries caused by increased intracranial pressure.

Conventionally, the study of neuronal cell death caused by hyperexcitation has long focused on the roles of sodium and calcium influx via the glutamate receptor channel. Recently, the importance of chloride influx has been suggested (see Non-Patent Publications 3-6). There have been attempts to realize clinical applications of medicines that are intended to inhibit hyperexcitation of neurons in brain ischemia and/or seizure. However, these medicines, which directly block the glutamate receptor, cause psychological abnormalities with serious side effects, which include unrest and hallucination (see Non-Patent Publication 7). There accordingly is an urgent need for the development of a novel inhibitor of neuronal hyperexcitation.
[Non-Patent Publication 1]
   Kimelberg HK. "Cunent concepts of brain edema" J Neurosurg. 83: 1051-1059 (1995)
[Non-Patent Publication 2]
   Griesdale DEG, Honer CR. "Aquaporins and brain edema" Surg. Neurol. 61: 418-421 (2004)
[Non-Patent Publication 3]
   Chen Q, Olney JW, Lukasiewicz PD, Almli T, Romano C. "Ca2+-independent excitotoxic neurodegeneration in isolated retina, an intact neural net: a role for Cl- and inhibitory transmitters" Mol. Pharmacol. 53: 564-572 (1998)
[Non-Patent Publication 4]
   Chen Q, Moulder K, Tenkova T, Hardy K, Olney JW, Romano C. "Excitotoxic cell death dependent on inhibitory receptor activation" Exp. Neurol. 160: 215-225 (1999)
[Non-Patent Publication 5]
   Burgos JS, Barat A, Ramirez G. "Cl--dependent excitotoxicity is associated with 3H2O influx in chick embryonic retina" NeuroReport 11: 3779-3782 (2000)
[Non-Patent Publication 6]
   Damme PV, Callewaert G, Eggermont J, Robberecht W, Bosch VD. "Chloride influx aggravates Ca2+-dependent AMPA receptor-mediated motoneuron death" J. Neurosci. 23: 4942-4950 (2003)
[Non-Patent Publication 7]
   Loescher W, Wlaz P, Szabo L. "Focal ischemia enhances the adverse effect potential of N-methyl-D-aspartate receptor antagonists in rats" Neuroscience Letters 240: 33-36 (1998)

The neuronal injury caused by hyperexcitation is observed not just in cerebrovascular disease such as brain tissue swelling (brain edema). It is commonly observed in various types of brain disorders, including ischemia and seizure. Inhibiting neuronal swelling and/or necrosis observed in an early stage of excitotoxic neuronal injury is very important to help prognosis of patients suffering from brain disorders.

The inventors of the present invention found that neuronal swelling caused by hyperexcitation activated volume-sensitive Cl- channels, and that the activity of volume-sensitive Cl- channels was associated with recovery of neurons to normal cell volume that occurs after removal of excitotoxic stimulation. Meanwhile, there is a possibility that the volume-sensitive Cl- channel activated by the hyperexcitation of the neurons would provide a pathway of chloride ion influx and aggravate cell swelling, which leads to cell rupture and eventually acute necrotic neuronal cell death. No one from this standpoint has attempted to develop a therapeutic method that is intended to inhibit (protection and/or relief against) neuronal swelling and/or necrosis caused by hyperexcitation.

Based on the foregoing findings, the present invention provides a method of protection or relief against excitotoxic neuronal injury (for example, neuronal swelling and/or necrosis caused by hyperexcitation). There is also provided a medicine for treating cerebrovascular disease caused by excitotoxic neuronal injury, through protection or relief against excitotoxic neuronal injury. Specifically, the present invention provides a method and medicine for protection and/or relief against excitotoxic neuronal injury, using Cl- channel blockers to regulate activity of the volume-sensitive Cl- channels that are activated during hyperexcitation of the neurons.

### DISCLOSURE OF INVENTION

A therapeutic agent according to the present invention includes a volume-sensitive Cl- channel blocker for treatment of vascular disease involving excitotoxicity.

In a therapeutic agent according to the present invention, it is preferable that the vascular disease be ischemic cerebrovascular disease.

In a therapeutic agent according to the present invention, it is preferable that the cells be neurons.

In a therapeutic agent according to the present invention, it is preferable that the cell injury occur during brain tissue swelling (brain edema), seizure, stroke, myocardial infarction, heart transplant, or brain vessel anastomosis.

In a therapeutic agent according to the present invention, it is preferable that the cell injury accompany severe cell swelling.

In a therapeutic agent according to the present invention, it is preferable that the cell injury accompany necrosis.

In a therapeutic agent according to the present invention, it is preferable that the cell injury accompany a significantly increased level of propidium iodide (PI) permeability to plasma membrane.

In a therapeutic agent according to the present invention, it is preferable that the cell injury accompany a significantly increased level of lactate dehydrogenase (LDH) release from cytoplasm.

In a therapeutic agent according to the present invention, it is preferable that the volume-sensitive Cl- channel blocker be NPPB or phloretin.

A therapeutic method according to the present invention, for treatment of a disease involving excitotoxicity, includes a step of applying a volume-sensitive Cl- channel blocker to cells.

In a therapeutic method according to the present invention, it is preferable that the disease be ischemic cerebrovascular disease.

In a therapeutic method according to the present invention, it is preferable that the cells be neurons.

In a therapeutic method according to the present invention, it is preferable that the cell injury occur during brain tissue swelling (brain edema), seizure, stroke, myocardial infarction, heart transplant, or brain vessel anastomosis.

In a therapeutic method according to the present invention, it is preferable that the cell injury accompany severe cell swelling.

In a therapeutic method according to the present invention, it is preferable that the cell injury accompany necrosis.

In a therapeutic method according to the present invention, it is preferable that the cell injury accompany a significantly increased level of propidium iodide (PI) permeability to plasma membrane.

In a therapeutic method according to the present invention, it is preferable that the cell injury accompany a significantly increased level of lactate dehydrogenase (LDH) release from cytoplasm.

In a therapeutic method according to the present invention, it is preferable that the volume-sensitive Cl- channel blocker be NPPB or phloretin.

An evaluation method for evaluating cell death according to the present invention is for evaluating cell death caused by hyperexcitation in an in *vitro* cell culture system, and includes: a first measuring step of measuring cell death in cultured cells; a stimulating step of subjecting the cells to excitotoxic stimulation; a second measuring step of measuring cell death in the cells subjected to excitotoxic stimulation; and a comparing step of comparing a first measurement value, obtained in the first measuring step, with a second measurement value, obtained in the second measuring step.

In an evaluation method for evaluating cell death according to the present invention, it is preferable that the cells be neurons.

In an evaluation method for evaluating cell death according to the present invention, it is preferable that the first measuring step and the second measuring step measure cell death using activity of mitochondrion dehydrogenase as a measure.

In an evaluation method for evaluating cell death according to the present invention, it is preferable, in the first measuring step and the second measuring step, that fragmentation of intranuclear DNA be used as a measure.

In an evaluation method for evaluating cell death according to the present invention, it is preferable, in the first measuring step and the second measuring step, that formation of varicosities in the cells be used as a measure.

It is preferable that an evaluation method for evaluating cell death according to the present invention further include a step of detecting Cl- current in varicosities.

It is preferable that an evaluation method for evaluating cell death according to the present invention further include a step of applying a volume-sensitive Cl- channel blocker to the cells after the first measuring step.

A screening method for screening a factor inhibiting cell injury according to the present invention is for inhibiting cell injury caused by hyperexcitation in an in *vitro* cell culture system, and includes: a first measuring step of measuring cell death in cultured cells; a step of applying a candidate factor to the cultured cells; a stimulating step of subjecting the candidate factor-applied cells to excitotoxic stimulation; a second measuring step of measuring cell death in the cells subjected to excitotoxic stimulation; and a comparing step of comparing a first measurement value, obtained in the first measuring step, with a second measurement value, obtained in the second measuring step.

In a screening method according to the present invention, it is preferable that the cells be neurons.

It is preferable that a screening method according to the present invention further include a step of applying a volume-sensitive Cl- channel blocker to the cells after the first measuring step.

An evaluation method for evaluating neuronal swelling according to the present invention is for evaluating neuronal swelling caused by hyperexcitation in an *in vitro* cell culture system, and includes: a stimulating step of subjecting cultured neurons to excitotoxic stimulation; a step of visualizing dendrites of the neurons; and a step of detecting formation of varicosities in the neurons.

In an evaluation method for evaluating neuronal swelling according to the present invention, it is preferable that the visualizing step employ EGFP gene transfection.

Additional objects, features, and strengths of the present invention will be made clear by the description below. Further, the advantages of the present invention will be evident from the following explanation in reference to the drawings.

### BRIEF DESCRIPTION OF DRAWINGS

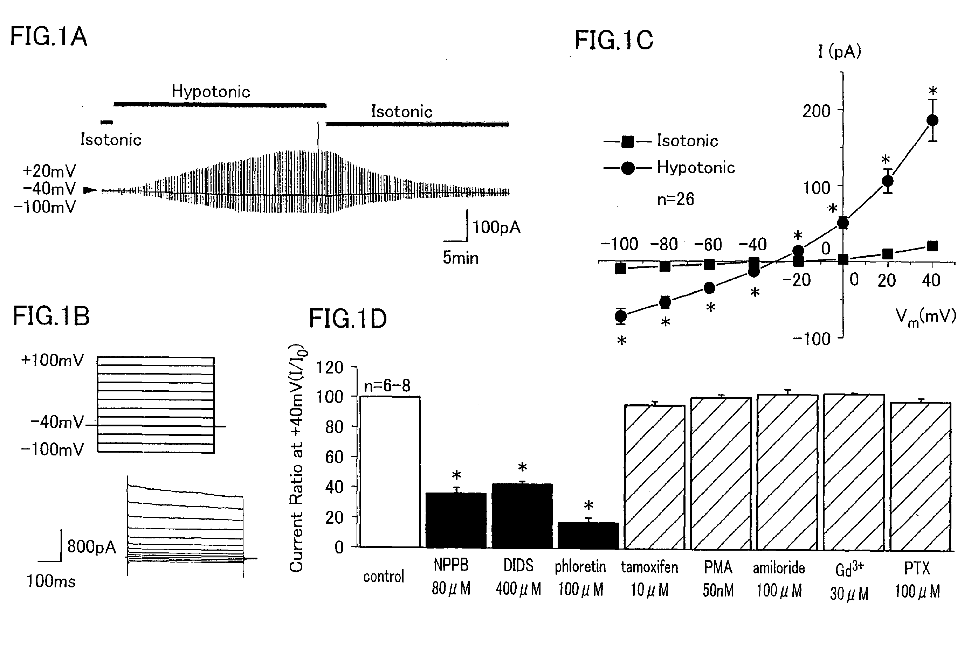

Figure 1 is a graph showing expression of voltage-sensitive Cl- channel in cerebral cortical neurons, in which A represents a graph showing the presence of voltage-sensitive Cl- channel in primary cultured cerebral cortical neurons as a result of whole-cell patch-clamp recordings; B represents a graph showing that the current observed in A exhibits inactivation at large positive potentials; C represent a graph showing, from the current-voltage curve, that the current observed in A exhibits outward rectification and that the depolarizing potential is close to the equilibrium potential of Cl-; and D represent a graph showing pharmacological characteristics of voltage-sensitive Cl- channel expressed in the cerebral cortical neurons.

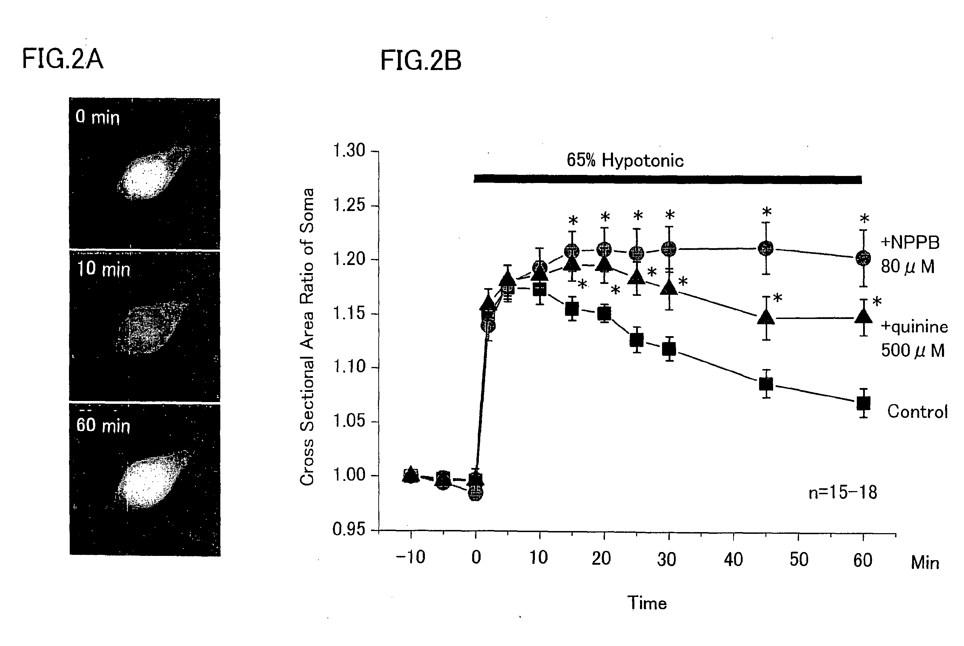

Figure 2 is a diagram showing involvement of voltage-sensitive Cl- channel in volume regulation of cerebral cortical neurons, in which A represents photographs showing X-Y cross sectional images of visualized calcein-AM-loaded primary cultured neurons on day 5-8 of culture, as observed over time with a two-photon laser microscope; and B is a graph representing a result of evaluation on cell size in the presence of various kinds of medicines, as observed based on changes in cross sectional area of the soma.

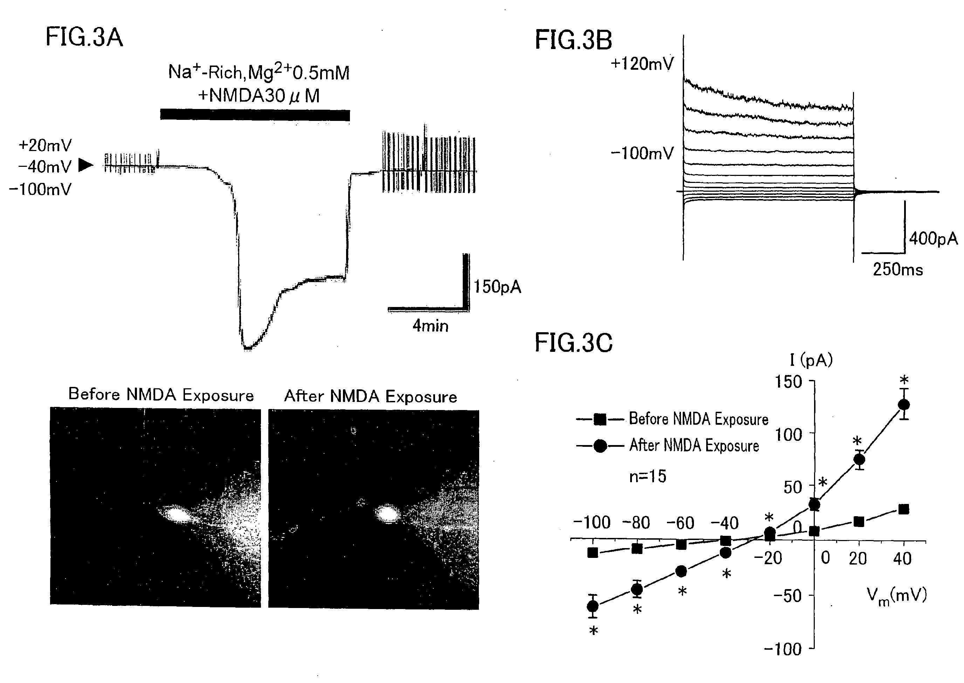

Figure 3 is a diagram showing activation of voltage-sensitive Cl- channel by excitotoxic stimulation, in which A represents a photograph and graph showing formation of varicosities on dendrites in cerebral cortical neurons exposed to excitotoxic stimulation, and activation of current after removal of agonist; B represents a graph showing that the activated current in B exhibits inactivation at large positive potentials; and C represents a graph showing, from the current-voltage curve, that the current observed in A exhibits outward rectification and that the depolarizing potential is close to the equilibrium potential of Cl⁻.

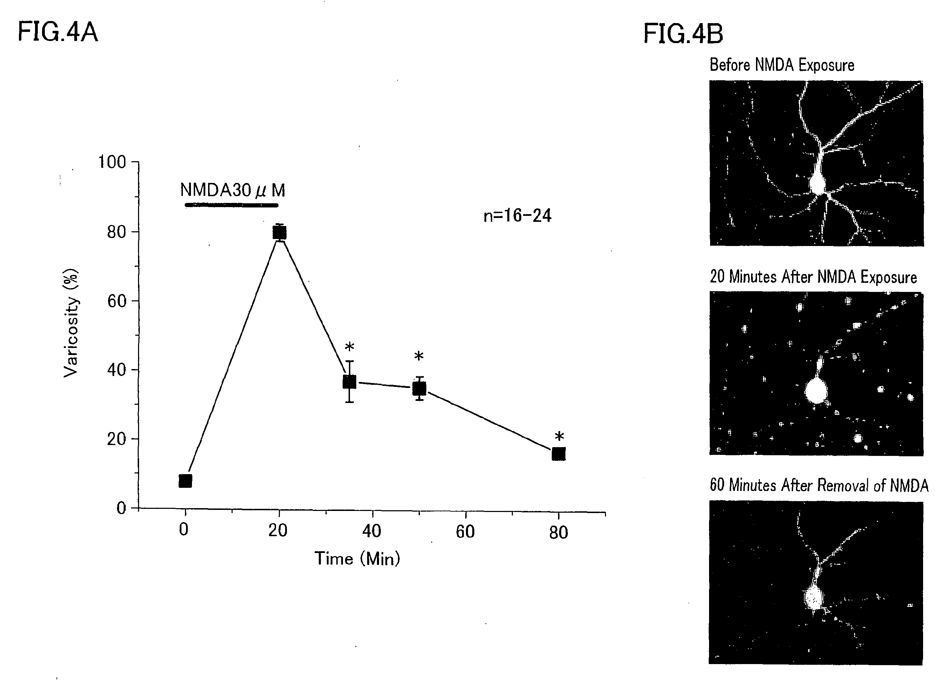

Figure 4A is a graph showing recovery from varicosities formed by excitotoxic stimulation, after removal of stimulation; and Figure 4B represents photographs showing recovery from varicosities formed by excitotoxic stimulation, after removal of stimulation.

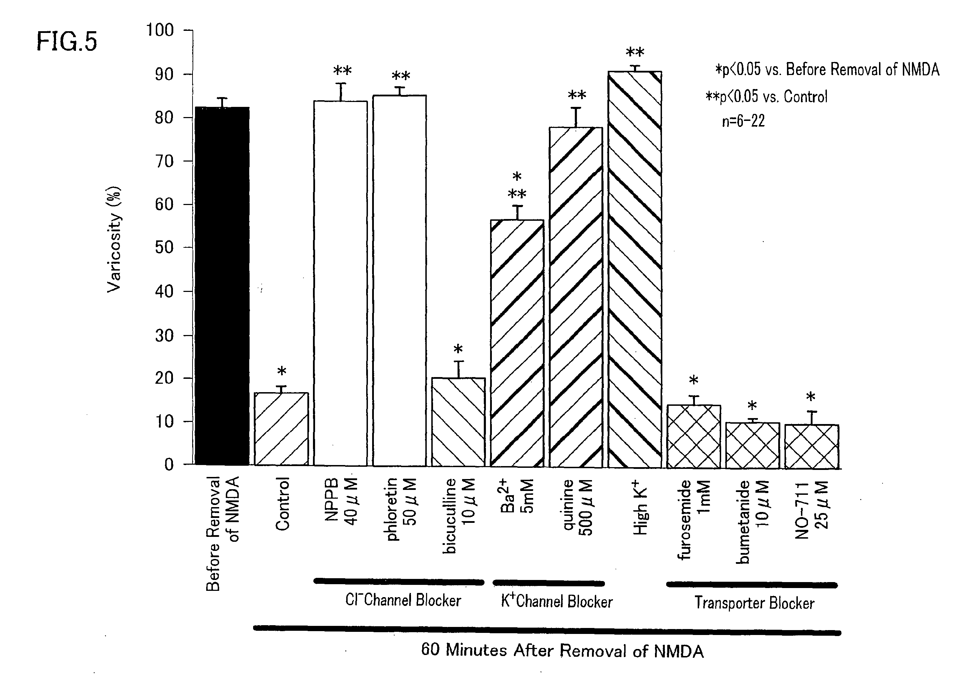

Figure 5 is a graph representing results of pharmacological identification of molecules involved in the recovery process from varicosities.

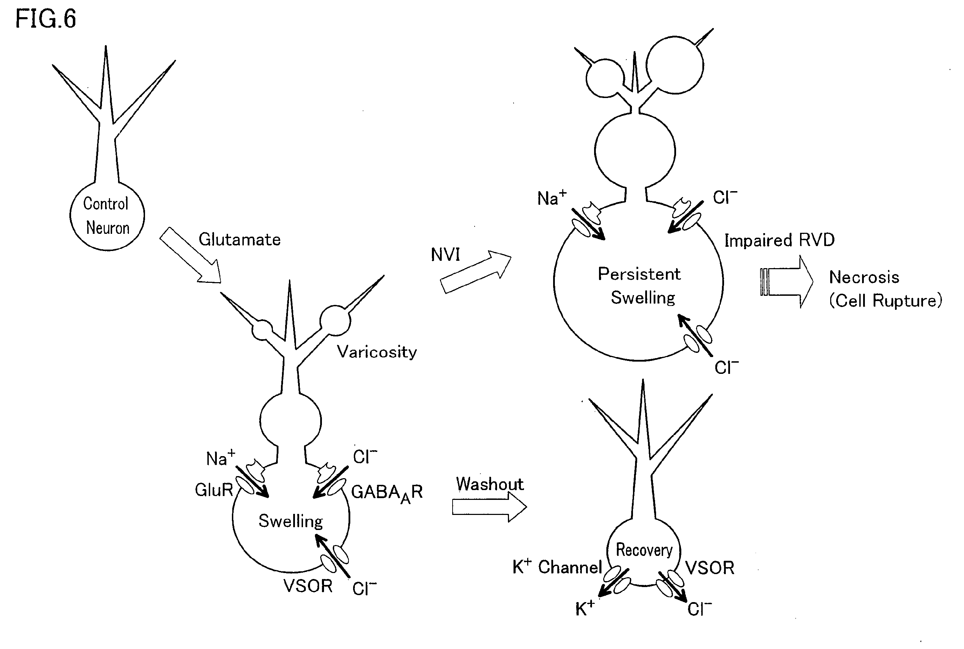

Figure 6 is a diagram conceptualizing effects of excitotoxic stimulation on neurons, and regulation by voltage-sensitive C1-channel.

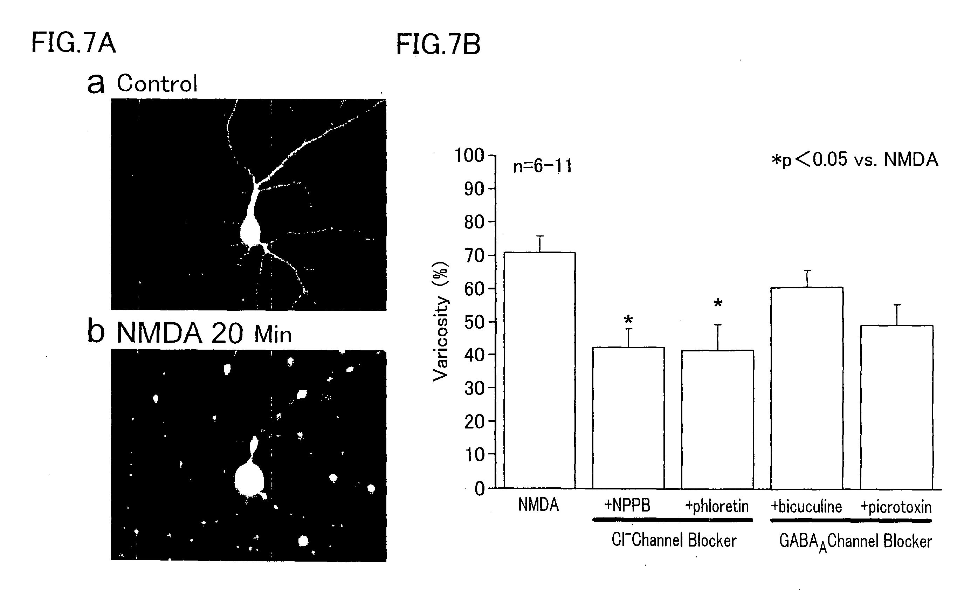

Figure 7 is a diagram representing inhibition of excitotoxic neuronal swelling by voltage-sensitive Cl- channel blocker; in which A represents micrographs showing excitotoxic neuronal swelling caused by an agonist of glutamate receptor; and B is a graph representing the inhibitory effects of the Cl- channel blocker on varicosity formation.

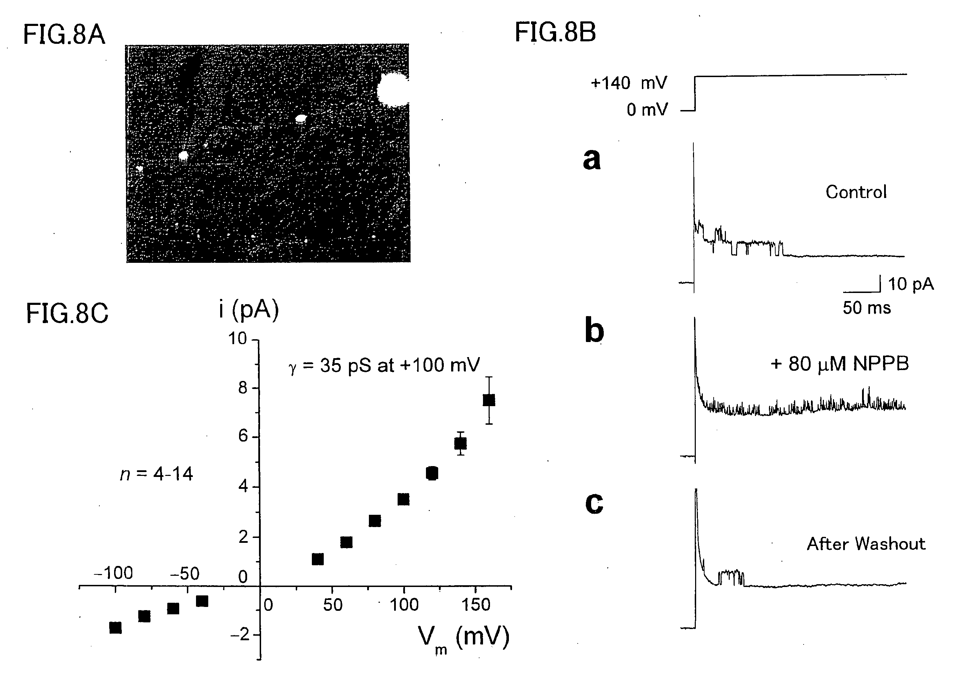

Figure 8A is a photograph showing how a patch-clamp experiment is conducted on varicosity membrane; Figure 8B is a graph representing unitary channel current records of volume-sensitive Cl⁻ channel (a is a control, b represents a result 3 minutes after application of NPPB, and c represents a result 3 minutes after removal of NPPB); C is a graph representing a unitary current-voltage curve of volume-sensitive Cl- channel.

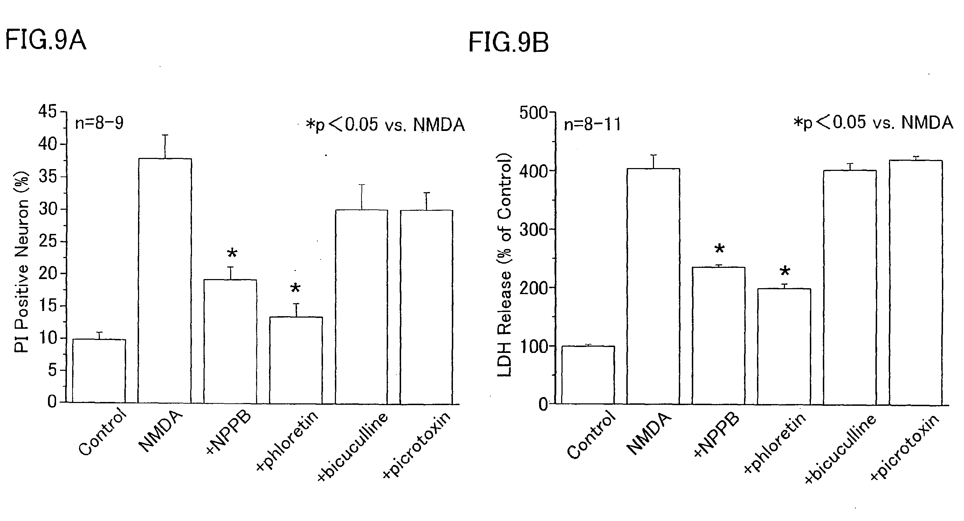

Figure 9 represents graphs showing inhibition of excitotoxic neuronal cell death by volume-sensitive C1- channel blocker; in which A represents a graph showing results of determining cell death by PI staining after NMDA exposure for 3 hours; and B represents a graph showing results of determining cell death by measuring LDH release after NMDA exposure for 3 hours.

### BEST MODE FOR CARRYING OUT THE INVENTION

Primary cultured mouse cerebral cortical neurons were exposed to an agonist for glutamate receptors to induce excitotoxic pathological conditions. The excitotoxicity and/or necrosis that occurred in the hyperexcited neurons were assessed based on morphological observation, examination of damage to plasma membrane, or measurement of LDH release. By studying effects of various medicines in these measurements, a screening method of medicines was established that is effective for treatment of or protection against excitotoxic neuronal injury that occurs during brain ischemia and/or seizure.

As a result of the foregoing assessments, the inventors of the present invention found that the neuronal necrosis caused by pathological hyperexcitation could be significantly suppressed when volume-sensitive Cl⁻ channel blockers were applied to neurons under excitotoxic conditions. It is envisaged that this necrosis-relieving mechanism is due to suppressed activity of the volume-sensitive Cl- channel associated with prolonged neuronal swelling that occurs during excitotoxic stimulation.

Based on the foregoing findings, the inventors of the present invention developed a novel method of protection against cerebrovascular disease, by targeting at volume-sensitive Cl⁻ channel activation, which is an early response of excitotoxic neuronal injury. This has led to the accomplishment of the present invention.

As used herein, the term "neurons" means neurons in living organisms, commercially available neuron cultures, and primary cultured neurons originating in brain removed from a living organism. As used herein, the term "cerebrovascular disease" includes diseases or disorders, such as cerebral thrombosis, embolic stroke, and brain hemorrhage, that involve disruption or shortage of blood supply (oxygen, nutrients, etc.) to tissues and/or cells that are under control of brain vessels. The term "neuronal injury" refers to the process in which necrotic or apoptotic cell death occurs as a result of disturbance in excitatory neuronal functions during stroke, brain edema, or seizure. The term "excitotoxic" refers to the condition in which neurons are overexcited by activation of the glutamate receptor channels in the neuronal cell membrane in response to release of glutamate from neurons or glial cells.

### (1) Therapeutic Agent and Therapeutic Method

In one aspect, the present invention provides a therapeutic agent for treating disorders involving excitotoxicity. As used herein, the term "excitotoxic" is used interchangeably with "caused by hyperexcitation." Further, as used herein, the term "treatment" concerns disorders, diseases, and injuries, and it refers to not only treatment of disorders, diseases, and injuries but also prevention, protection, and relief, as well as inhibition, alleviation, and improvement of symptoms or conditions of disorders, diseases, and injuries.

In the present embodiment, the disorder is preferably an ischemic cerebrovascular disease. In the present embodiment, the cells are preferably neurons. A therapeutic agent according to the present invention preferably includes Cl- channel blockers. In a preferred embodiment, the Cl- channel is a volume-sensitive Cl- channel. In a more preferred embodiment, the Cl- channel blockers comprise NPPB or phloretin.

As described above, hyperexcitation of neurons causes neuronal swelling. This results in (1) increase in intracranial pressure as the neuronal swelling exerts pressure on surrounding cells or entire brain tissues, and (2) disruption of the neurons (i.e., necrosis). The inventors of the present invention found that the early response of the excitotoxic neuronal injury was the activation of the volume-sensitive C1-channel, and successfully developed protection and/or relief against excitotoxic neuronal injury by inhibiting such early response with the Cl- channel blocker.

Non-limiting examples of a cerebrovascular disease involving excitotoxic neuronal injury include brain tissue swelling (brain edema), seizure, stroke, and myocardial infarction.

Cell death can be either necrosis or apoptosis. As used herein, the term "necrosis" is used interchangeably with "cell death caused by necrosis" or "necrotic cell death." The term "apoptosis" is used interchangeably with "cell death caused by apoptosis" or "apoptotic cell death." An evaluation method of cell death will be described later.

A therapeutic agent according to the present invention is applicable to mammals such as humans, mice, rats, rabbits, dogs, cats, cattle, horses, pigs, and monkeys.

A therapeutic agent according to the present invention may solely comprise a Cl- channel blocker, but may additionally include a pharmaceutically acceptable carrier. A therapeutic agent according to the present invention can be produced according to known means in producing methods of medical compositions. A pharmaceutically acceptable carrier used in such medical compositions can be selected according to administration form or dosage form of the medical composition.

As used herein, pharmacologically acceptable carriers are various kinds of organic and inorganic carrier substances that can be used as material of the drug. Such carriers are contained as a diluting agent, a lubricant, a binder, or a disintegrant in the case of solid drugs. In liquid drugs, the carriers are contained as a solvent, a solubilizing agent, a suspension, an isotonic agent, a buffer, or a soothing agent.

Examples of a diluting agent include: lactose, saccharose, D-mannitol, xylitol, sorbitol, erythrytol, starch, and crystallized cellulose. Examples of a lubricant include: magnesium stearate, calcium stearate, talc, and colloid silica.

Examples of a binder include: pregelatinized starch, methyl cellulose, crystallized cellulose, saccharose, D-mannitol, trehalose, dextrin, hydroxypropylcellulose, hydroxypropyl methylcellulose, and polyvinyl pyrrolidone.

Examples of a disintegrant include: starch, carboxymethyl cellulose, low-substitution hydroxypropylcellulose, carboxymethylcellulose calcium, croscarmellose sodium, and sodium carboxymethyl starch.

Examples of a solvent include: injection solvent, alcohol, propylene glycol, macrogol, sesame oil, corn oil, and tricaprylin.

Examples of a solubilizing agent include: polyethylene glycol, propylene glycol, D-mannitol, trehalose, benzyl benzoate, ethanol, tris aminomethane, cholesterol, triethanolamine, sodium carbonate, and sodium citrate.

Examples of a suspension include: detergents such as stearyl triethanolamine, sodium lauryl sulfate, lauryl amino propionic acid, lecithin, benzalkonium chloride, benzethonium chloride, and glyceryl monostearate; and hydrophilic high-molecular-weight molecules such as polyvinyl alcohol, polyvinyl pyrrolidone; carboxymethylcellulose sodium, methyl cellulose, hydroxymethyl cellulose, hydroxyethyl cellulose, and hydroxypropylcellulose.

Examples of an isotonic agent include: sodium chloride, glycerine, and D-mannitol.

Examples of a buffer include: phosphate, acetate, carbonate, and citrate.

Examples of a soothing agent include benzyl alcohol.

Examples of an antiseptic include: parahydroxybenzoate esters, chlorobutanol, benzyl alcohol, phenethyl alcohol, dehydroacetic acid, and sorbic acid.

Examples of an antioxidant include sulfite salt and ascorbic acid.

A medical composition according to the present embodiment can be produced according to a method commonly used in the field of drug manufacturing. The content of C1-channel blocker in a medical composition according to the present embodiment is not particularly limited as long as the Cl- channel blocker can be administered in a specified dose (described later) with the medical composition, taking into account form and method of administration, etc.

A medical composition according to the present embodiment may be either orally administered in the form of, for example, a tablet, a capsule (including a soft capsule and a micro capsule, for example), a powder, a granule, or syrup, or non-orally administered in the form of, for example, injection, suppository, pellet, or drip infusion. A therapeutic agent according to the present invention low in toxicity, and therefore the medical composition can be administered either orally or non-orally. As used herein, the term "non-orally" refers to form of administration, including intraventricular injection or infusion, intravenous injection or infusion, intramuscular injection or infusion, intra-abdominal injection or infusion, intrasternal injection or infusion, subcutaneous injection or infusion, and intra-articular injection or infusion.

A dose of a medical composition according to the present embodiment varies depending on such factors as type of subject, administration route, and symptoms. A person ordinary skill in the art will be able to suitably set an optimal condition according to these factors.

A medical composition according to the present embodiment can be administered in an administration route as suitably selected according to form of the drug. The method of administration is not particularly limited, and it can be made either internally or externally, or by injection. The injection may be made intraventricularly, intravenously, intramuscularly, subcutaneously, or intradermally, for example.

A dose of a medical composition according to the present embodiment varies depending upon such factors as form of drug, method of administration, intended use, or age, weight, and symptoms of a patient exposed to the medical composition, and as such a dose is suitably set according to these factors. Generally, a daily dose of 5 to 400 µg/kg is preferable for adults in terms of an amount of active ingredient contained in the drug. However, since the dose varies depending on various conditions, a dose below or above this range may be sufficient or needed to exhibit the effects. Administration may be made either at once or in separate doses in a day, within a predetermined dose range. Further, a medical composition according to the present embodiment may be orally administered either directly or with any food or drinks in a patient's daily diet.

In another aspect, the present invention provides a therapeutic method for treating a cerebrovascular disease involving excitotoxic neuronal injury. A therapeutic method according to the present invention preferably includes a step of administering a Cl- channel blocker to neurons. In a therapeutic method according to the present invention, a medicine according to the present invention is used as the Cl- channel blocker. By administration of a therapeutic agent according to the present invention, the early response of hyperexcitotatory neuronal injury can be inhibited.

In other words, a therapeutic agent according to the present invention at least includes a Cl- channel blocker. As such, a therapeutic agent including different kinds of C1-channel blockers also falls within a technical scope of the present invention. A therapeutic method according to the present invention includes a step of applying at least a C1-channel blocker. As such, a therapeutic method applying a plurality of Cl- channel blockers also falls within a technical scope of the present invention.

Further, the present invention is based on the finding that the volume-sensitive Cl- channel blocker inhibits neuronal necrosis caused by hyperexcitation. As such, a technical scope of the present invention also encompasses a necrosis inhibitor including a volume-sensitive Cl- channel blocker, a necrosis inhibiting method using a volume-sensitive Cl- channel blocker, and a necrosis inhibiting kit provided with a volume-sensitive Cl- channel blocker.

### (2) Evaluation Method of Cell Death Caused by Hyperexcitation

The present invention provides an evaluation method for evaluating cell death caused by hyperexcitation in an in *vitro* cultured cell system. An evaluation method of cell death according to the present invention includes a measuring step of measuring cell death in cultured cells, and a stimulating step of subjecting the cells to excitotoxic stimulation. In one embodiment, it is preferable that the measuring step of cell death be performed before and after the stimulating step, and more preferably the method further includes a comparing step of comparing a first measurement value, obtained in a first measuring step performed before the stimulating step, and a second measurement value, obtained in a second measuring step performed after the stimulating step. In the present embodiment, it is preferable in the stimulating step that the cultured cells be supplemented with a buffer (medium) serving as an excitotoxic stimulant of the cultured cells.

In an evaluation method of cell death according to the present invention, the tested cells are subjected to excitotoxic stimulation. Thus, an in *vitro* evaluation method of cell death according to the present invention enables evaluation of cell death based on, for example, cell viability, before and after excitotoxic stimulation. By comparing results of evaluation, excitotoxic cell death can be easily evaluated.

In an evaluation method of cell death according to the present invention, the type of tested cells is not particularly limited. For example, cardiomyocytes, neurons, brain glial cells may be used. These cells may be commercially available cultured cells or primary cultured cells. Primary cultured cerebral cortical neurons are most preferable. Primary cultured cerebral cortical neurons allows for evaluation of neuronal cell death (preferably, necrosis) caused by excitotoxic stimulation.

The buffer used in the stimulating step is preferably, for example, EBSS containing 30 µM N-methyl-D-aspartate (NMDA) and 30 µM glycine, supplemented with 2 mM CaCl₂ and 0.5 mM MgCl₂.

For the evaluation of cell swelling, the duration of stimulation in the stimulating step is preferably 10 to 20 minutes, or most preferably 20 minutes. For the evaluation of cell death, 90 to 240 minutes is preferable, and 180 minutes is most preferable. The culture temperature in the culturing step and the stimulating step is preferably 37°C.

The step of detecting cell death is not particularly limited. For example, detection of cell death may be made based on activity of mitochondrion dehydrogenase (MTT method or MTS method), fragmentation of intranuclear DNA (DNA ladder test), activity of cell death-causing intracellular protease (caspase), release of cytochrome c from mitochondrion into cytoplasm, or changes in morphology of the nucleus (for example, condensation or fragmentation of the nucleus).

Cell death accompanies a reduction in the activity of mitochondrion dehydrogenase, and therefore a reduced dehydrogenase activity can be regarded as an increased cell death rate, using activity of mitochondrion dehydrogenase as a measure.

Cell death caused by necrosis involves membrane injury in the cells, allowing the cells to be stained with propidium iodide (PI). By staining the nucleus of all cells with Hoechst 33342, the proportion of cells that have undergone necrotic cell death can be calculated.

Cell death caused by apoptosis involves fragmentation of intracellular DNA. Thus, by observing a state of intracellular ladder-like DNA by electrophoresis for example, it is possible to readily determine whether the cell death is attributed to apoptotic cell death.

Further, since cell death caused by apoptosis is known to activate caspase (raise activity of the enzyme), an increase in enzyme activity can be regarded as an increased cell death rate in apoptotic cell death, using caspase activity as a measure.

Further, in apoptotic cell death induced by stimulation of mitochondria, the mitochondria release cytochrome c into the cytoplasm. This can be used as a measure of cell death. By measuring the quantity of cytochrome c released into the cytoplasm, it is possible to readily determine whether the cell death is attributed to apoptotic cell death.

Further, apoptotic cell death involves changes in morphology of the nucleus, such as condensation or fragmentation. This can also be used as a measure of cell death. By observing changes in morphology of the nucleus with an electron microscope for example, it is possible to readily determine whether the cell death is attributed to apoptotic cell death. When neurons are used, hyperexcitation of the neurons generates varicosities in the dendrites. Excitotoxic cell death can also be detected using formation of varicosities as a measure. By detecting Cl- current in the varicosities, it is possible to examine whether the excitotoxic cell death involves the Cl- channel (preferably, volume-sensitive Cl- channel). Here, involvement of Cl- channel in the excitotoxic cell death can be examined more accurately by pre-applying Cl- channel blockers to the cells.

By performing these cell-death detecting steps either individually or in combination, it is possible to determine whether the induced cell death is necrosis or apoptosis.

### (3) Screening Method of Factors Inhibiting Excitotoxicity

An evaluation method of cell death according to the present invention can be effectively used to provide a medicine that inhibits excitotoxic cell death, and in particular, for screening of a medicine that inhibits excitotoxic cell death. Specifically, by comparing results of cell death evaluation performed before and after the stimulating step, the inhibitory effect of the candidate factor against the excitotoxic cell death can be evaluated. The result of evaluation can then be used to provide a substance having inhibitory effects against the excitotoxic cell death. The present invention provides a screening method for screening factors inhibiting excitotoxicity in an in *vitro* cell culture system.

As with the evaluation method, a screening method according to the present invention includes a measuring step of measuring cell death in cultured cells, and a stimulating step of subjecting the cells to excitotoxic stimulation. In one embodiment, it is preferable that the measuring step of cell death be performed before and after the stimulating step, and more preferably the method further includes a comparing step of comparing a first measurement value, obtained in a first measuring step performed before the stimulating step, and a second measurement value, obtained in a second measuring step performed after the stimulating step. In the present embodiment, it is preferable in the stimulating step that the cultured cells be supplemented with a buffer (medium) serving as an excitotoxic stimulant of the cultured cells. A screening method according to the present embodiment further includes the step of applying a candidate factor to the cells after the first measuring step (before the stimulating step).

By further including the step of applying a Cl- channel blocker to the cells after the first measuring step, a screening method according to the present invention can screen for a target factor inhibiting cell injury involving the Cl- channel (preferably, volume-sensitive Cl⁻ channel).

A screening method according to the present invention can be used to easily screen for factors inhibiting cell injury induced by excitotoxic stimulation.

### (4) Evaluation Method of Neuronal Swelling Caused by hyperexcitation

The present invention provides a method for evaluating neuronal swelling caused by hyperexcitation in an in *vitro* cell culture system.

An evaluation method of neuronal swelling according to the present invention includes a stimulating step of subjecting cultured neurons to excitotoxic stimulation, a step of visualizing dendrites of the neurons, and a step of detecting formation of varicosities in the neurons.

For the step of visualizing dendrites of the neurons, various kinds of techniques known in the art can be used (for example, transfection of cells with EGFP gene).

In an evaluation method of neuronal swelling according to the present invention, the tested cells are subjected to excitotoxic stimulation. Thus, an evaluation method of neuronal swelling according to the present invention enables easy evaluation of excitotoxic neuronal swelling, by comparing varicosities before and after excitotoxic stimulation.

An evaluation method of neuronal swelling according to the present invention can use the same cells and the same buffers for the stimulating step used in the evaluation method of cell death.

An evaluation method of neuronal swelling according to the present invention is effective for screening of a medicine inhibiting neuronal swelling induced by hyperexcitation. Specifically, by comparing results of cell death evaluation performed before and after the stimulating step, it is possible to evaluate the inhibitory effect of the candidate factor against the neuronal swelling caused by hyperexcitation. The result of evaluation can then be used to provide a substance having inhibitory effects against neuronal swelling caused by hyperexcitation.

The following will describe the present invention in more detail by way of Examples. It should be appreciated however that the present invention is not limited in any way by the following description.

### [Example 1: Expression of Volume-Sensitive Cl- channel in Cerebral Cortical Neurons]

The cerebral cortex of fetuses removed from mice on day 16 of pregnancy was plated on cover-glass slips after digestion with papain. To prevent growth of glias and other proliferative cells, the cultures were supplemented with AraC (4 µM) 2 days after plating and a neuron-rich (>95%) culture system was prepared. A whole-cell patch-clamp technique was applied to primary cultures of cerebral cortical neurons (5-8 days after incubation). The intracellular and extracellular solutions used for the patch-clamp experiments were composed of the following. Intracellular solution (300mOsm, pH 7.4): 24 mM NMDG-C1, 100 mM NMDG-aspartate, 2 mM MgCl₂, 5 mM Na₂ATP, 0.3 mMNa₃ GTP, 1 mM EGTA, 10 mM HEPES. Extracellular solution (iso-osmotic solution 300mOsm, hyposmotic solution 270mOsm, pH7.4): 124 mM NMDG-Cl, 10 mM NaCl, 4 mM MgCl₂, 0.33 mM NaH₂PO₄, 0.5 mM EGTA, 2 mM 4-aminopyridine (4-AP), 10 mM HEPES, 5.5 mM glucose. Osmolality was adjusted by adding mannitol. Current activation was observed overtime by applying a potential of ±60 mV (500 ms duration, every 15 s) from a holding potential of -40 mV. To observe current kinetics, step pulses from -100 mV to +100 mV in 20 mV increments were applied (0.5 s duration) and current response was observed. The recordings were plotted in a current-voltage curve. The result is shown in Figure 1.

The whole-cell patch-clamp recording revealed the presence of volume-sensitive Cl- channels in the primary cultured cerebral cortical neurons. The cerebral cortical neurons exhibited gradually activated current by hyposmotic stimulation (A). This current exhibited inactivation at large positive potentials (B). It can be seen from the current-voltage curve that the activated current exhibits outward rectification and that the depolarizing potential is close to the equilibrium potential of Cl- (C). The fact that this potential was inhibited by NPPB (80 µM), DIDS (400 µM), and phloretin (100 µM) (D) demonstrates that the cerebral cortical neurons in fact express the C1⁻ channel, or volume-sensitive Cl- channel, activated by hyposmitic stimulation.

### [Example 2: Involvement of Volume-Sensitive Cl- Channel in Volume Regulation of Cerebral Cortical Neurons]

Primary cultured neurons on day 5-8 of culture were loaded with calcein-AM to visualize the cells, and X-Y cross sectional images were captured over time with a two-photon laser microscope. Cell size was assessed based on changes in cross sectional area of the soma. An area ratio at each time point was calculated and plotted on a graph, with the area at the start of experiment given as 1 (Figure 2B). The solution used for the experiment had the following composition: 90 mM NaCl, 2.5 mM KCl, 5 mM HEPES, 2 mM MgCl₂, 2 mM CaCl₂, and 10 mM glucose. Osmolality was adjusted by adding mannitol: 315mOsm (iso-osmotic solution) and 200mOsm (hyposmotic solution). The result is shown in Figure 2.

The cerebral cortical neurons can recover from swelling under hypotonic conditions and restore the original volume (solid lines A, B). The fact that the volume regulation is inhibited by quinine (500 µM) and NPPB (80 µM) indicates involvement of K⁺ channels, sensitive to quinine, and volume-sensitive Cl- channels, sensitive to NPPB (B).

### [Example 3: Activation of Volume-Sensitive Cl- channel by Excitotoxic Stimulation]

A whole-cell patch-clamp technique was applied to primary cultured neurons on day 5-8 of culture. The cells for whole-cell clamp recordings were subjected to an excitotoxic stimulant by 10-minute exposure to low-magnesium buffer (composition: 142 mM Nacl, 2 mM 4-AP, 0.5 mM EGTA, 0.5 mM MgCl₂, 0.33 mM NaH₂PO₄, 10 mM HEPES, 5.5 mM glucose, 0.03 mM glycine) containing NMDA (30 µM). Currents before and after excitotoxic stimulation were compared. During current recordings, 5 µM calcein was added to the internal solution to observe cell morphology. The result is shown in Figure 3.

Application of excitotoxic stimulation to the cerebral cortical neurons accompanied formation of varicosities on the dendrites (A, right). Current activation was exhibited after removal of the agonist (A). The activated current exhibited inactivation at large positive potentials (B). The current-voltage curve showed outward rectification, and the depolarizing potential was close to the equilibrium potential of Cl-. It was therefore demonstrated that this channel had the same properties as the volume-sensitive Cl- channel.

### [Example 4: Recovery from Varicosities after Removal of Excitotoxic stimulation]

Primary cultured cerebral cortical neurons were transfected on day 3 of culture with a plasmid containing green fluorescent protein EGFP gene, using a potassium phosphate method. On the next day, GFP expression was observed in <0.1% of neurons. Cells on day 2-6 of transfection were used for the experiment.

The cells were subjected to an excitotoxic stimulant by 20-minute exposure to low-magnesium HEPES buffer (composition: 142 mM NaCl, 2.5 mM KCl, 0.5 mM EGTA, 0.5 mM MgCl₂, 0.33 mM NaH₂PO₄, 10 mM HEPES, 5.5 mM glucose, 0.03 mM glycine) containing NMDA (30 µM). After stimulation, the cells were washed three times with normal HEPES buffer (composition: 140 mM NaCl, 2.5 mM KCl, 2 mM CaCl₂, 2 mM MgCl₂, 10 mM HEPES, 5.5 mM glucose, 35 mM mannitol). The cells were then incubated at 37°C in the same buffer, and fixed with 4% paraformaldehyde at different time points (0, 15, 30, 60 min). After fixing, the morphology of GFP-expressing neurons was observed with a fluorescent microscope, and the number of cells forming varicosities (diameter >3 µm) on the dendrites, or "varicosity-bearing cells," was counted as a percentage of the total number of counted neurons.

### [Example 5: Pharmacological Identification of Molecule Involved in Recovery Process from Varicosities]

As in Example 4, EGFP-transfected primary cultured cerebral cortical neurons were used. According to the procedure of Example 4, the cells were subjected to excitotoxic stimulation, and incubated with normal HEPES buffer supplemented with various kinds of medicines. After 60 minutes, the cells were fixed with 4% paraformaldehyde, and the number of varicosity-bearing cells was counted with a fluorescent microscope as a percentage of the total number of counted neurons. The result is shown in Figure 5.

A pharmacological study was made as to which channel or transporter was involved in the recovery process from varicosities described with reference to Figure 4. Recovery from varicosities was significantly inhibited with the volume-sensitive Cl- channel blockers, NPPB and phloretin. The inhibitory effect was also observed with K⁺ channel blocker.

These results suggest that co-activation of Cl- channel and K⁺ channel plays a requisite role in recovery from varicosities after excitotoxic stimulation.

As conceptually illustrated in Figure 6, the excitotoxic stimulation by glutamate causes neurons to swell by the glutamate receptor-mediated Na⁺ influx and the Cl- influx mediated by unknown pathways (GABA_{A} channel and volume-sensitive Cl- channel are shown as candidate pathways in the Figure). The neurons undergo necrosis when exposed to stimulation for extended time periods, whereas the cell volume is restored when the stimulation is removed in a short time period. The volume-sensitive Cl⁻ channel plays an important role in this recovery process.

In the example below, primary cultured mouse cerebral cortex neurons were used to assess:
(1) whether the volume-sensitve Cl- channel blocker is effective for neuronal swelling caused by hyperexcitation;
(2) whether activation of volume-sensitive Cl- channel is detected on the dendrites of the neurons that have swelled by hyperexcitation; and
(3) whether the necrosis of neurons caused by hyperexcitation is inhibited by application of volume-sensitive Cl- channel blocker.

In order to access effects of volume-sensitive Cl- channel blocker on neuronal swelling or necrosis caused by hyperexcitation, primary cultures of cerebral cortical neurons isolated from fetuses removed from mice on day 16 of pregnancy were used. The primary cultured cerebral cortical neurons were cultured in a 5% CO₂ incubator at 37°C, using Dulbecco's medium supplemented with 10% fetal bovine serum, streptomycin, and penicillin. To visualize dendrites, the cultured cerebral cortical neurons were transfected with EGFP gene on day 3 of culture, using a potassium phosphate method. As an excitotoxic stimulant, Earle's Balanced Salt Solution (EBSS; commercially available as GIBCO#14155-063) was used that contained 30 µM N-methyl-D-aspartate (NMDA) and 30 µM glycine, supplemented with 2 mM CaCl₂ and 0.5 mM MgCl₂. To confirm activation of volume-sensitive Cl- channel, an experiment was conducted using a perforated-vesicle patch-clamp technique. After forming a gigaohm seal on the membrane of the varicosities exposed to excitotoxic stimulation, recording electrodes were raised to record currents from a single varicosity patch membrane. The intracellular and extracellular solutions used for the patch-clamp experiment were composed of the following. Extracellular solution: 124 mM N-methyl-D-glucamine (NMDG), 124 mM HCl, 20 mM TEA-Cl, 4 mM MgCl₂, 0.33 mM NaH₂PO₄, 0.5 mM EGTA, 10 mM 2-[4-(2-hydroxyethyl)-1-piperazinyl] ethanesulfonic acid (HEPES), 2 mM 4-aminopyridine (4-AP), 5.5 mM glucose, 270mOsm, pH7.4. Intracellular solution: 145 mM KCl, 10 mM HEPES, 1 mM EGTA, 1 mM MgCl₂, 0.3 mg/ml nystatin, 300mOsm, pH 7.4.

Neuronal swelling was assessed as a proportion of varicosity-forming cells in the total number of neurons. After 20-minute excitotoxic stimulation, the cells were fixed with 4% paraformaldehyde, and the number of cells in each cell group was counted with a fluorescent microscope.

Necrotic neuronal cell death was assessed by PI staining in which the nuclei are stained with a propidium iodide (PI) dye that enters the cells through damaged portions of the plasma membrane, or by measuring the level of extracellular lactate dehydrogenase (LDH) released from dead cells.

### [Example 6: Inhibitory Action of Volume-Sensitive Cl- Channel Blocker on Excitotoxic Swelling of Cerebral Cortical Neurons]

Figure 7A is micrographs showing excitotoxic swelling of neurons induced by glutamate receptor agonist (30 µM NMDA). Primary cultured cerebral cortical neurons were visualized through EGFP expression. In the Figure, a is a control, and b shows neurons 20 minutes after NMDA exposure. Figure 7B represents inhibitory action of Cl- channel blocker on varicosity formation. In the Figure, asterisk indicates a level of significant difference (p<0.05) from data on NMDA (before application of blocker).

The excitotoxic neuronal swelling requires not only sodium influx through glutamate receptors (having cation channels) but inflow of electrically obliged chloride. In 20 minutes of pathological excitotoxic stimulation, formation of varicosities (Figure 7Ab) as a measure of cell swelling was observed in about 70% of dendrites (Figure 7Aa) of the cerebral cortical neurons (the first bar in Figure 7B). The number of varicosity-forming neurons as a measure of neuronal swelling decreased significantly by application of volume-sensitive C1-channel blocker (40 µM NPPB or 100 µM phloretin) during excitotoxic stimulation (Figure 7B, the second and third bars). There were no significant inhibiting effects, though slight effects were observed, in application of the blocker (10 µM bicuculline or 100 µM picrotoxin) of the GABA_{A} receptors (having C1-channels), which generally serve as main Cl- pathways in neurons (Figure 7B, the fourth and fifth bars).

By NMDA-exposure, the neurons exhibited severe swelling that involved focal swelling of dendrites (formation of varicosities). The neuronal swelling as measured by formation of varicosities was significantly inhibited by application of volume-sensitive Cl- channel blocker (40 µM NPPB or 100 µM phloretin) during NMDA exposure. There was no statistically significant difference in the effects of GABA_{A} receptor C1-channel blocker (10 µM bicuculline or 100 µM picrotoxin).

### [Example 7: Activation of Volume-Sensitive Cl- Channel on Varicosity Membrane of Cerebral Cortical Neurons Swelled by Hyperexcitation]

It was confirmed whether the varicosity as a focal swelling of dendrites caused by pathological hyperexcitation had activation of volume-sensitive α-channels on its membrane (Figure 8).

Figure 8A is a micrograph showing a varicosity with a gigaohm seal formed thereon with a patch pipette. The patch pipette (shadow on upper left) was gigasealed on a varicosity formed on the dendrites extending from the soma (upper right). The patch pipette was then lifted up to form a perforated vesicle patch. Figure 8B represents unitary channel current records of volume-sensitive Cl- channel from varicosities formed by NMDA exposure. Recordings were performed with a varicosity membrane isolated by a perforated-vesicle patch technique. In the Figure, a is a control, b shows the result 3 minutes after NPPB application, and c shows the result 3 minutes after removal of NPPB. The panel on the top is the protocol of applied voltage steps. Figure 8C represents a unitary current-voltage curve of volume-sensitive Cl- channel on varicosities.

After forming a gigaohm seal on the membrane of varicosities formed by excitotoxic stimulation (Figure 8A), the recording electrodes were lifted up to excise a single varicosity, with which recordings of volume-sensitive Cl- currents were made (Figure 8B). The single-channel events observed on the varicosity were inactive at large positive potentials (Figure 8Ba), and were blocked by addition of 80 µM NPPB (Figure 8Bb). That is, the channels were inactive at large positive potentials (+140 mV), and the activity was inhibited with 80 µM NPPB. The blocking effect of NPPB abolished after removal (Figure 8Bc) of NPPB, showing that the effect was reversible. The unitary channel current (i) exhibited outward rectification (Figure 8C). The single-channel conductance (y) was of an intermediate type (35 pS at +100 mV) (Figure 8C). These properties of the recorded currents coincided with those of the volume-sensitive Cl- channel. This revealed that the volume-sensitive Cl- channel was indeed expressed and activated on the dendrites of neurons that had swelled by hyperexcitation.

### [Example 9: Relief of Excitotoxic Neuronal Cell Death with C1⁻ Channel Blocker]

Prolonged neuronal swelling by excitotoxic stimulation causes necrotic cell death in the form of cell rupture. Assessment was made as to whether application of volume-sensitive Cl- channel blocker during excitotoxic insult would also inhibit necrotic neuronal cell death as it inhibits cell swelling (Figure 9).

Figure 9A shows results of determining cell death by PI staining after NMDA exposure for 3 hours. Figure 9B shows results of determining cell death by measuring LDH release after NMDA exposure for 3 hours.

After excitotoxic stimulation for 3 hours, the proportion of PI-stained cells as a measure of damage to plasma membrane increased to about 40% of the total number of cells (Figure 9A, the second bar). By co-application of volume-sensitive C1-channel blocker (40 µM NPPB or 100 µM phloretin), the number of PI positive cells decreased significantly (Figure 9A, the third and fourth bars). There were no significant inhibiting effects, though slight effects were observed, in application of the C1⁻channel blocker (10 µM bicuculline or 100 µM picrotoxin) of the GABA_{A} receptors (Figure 9A, the fifth and sixth bars). As another measure of necrotic cell death, the level of LDH released into the extracellular domain was measured (Figure 9B). In neurons that had co-application of volume-sensitive C1-channel blocker (40 µM NPPB or 100 µM phloretin) during excitotoxic insult, the level of LDH release was significantly inhibited (Figure 9B, the third and fourth bars) as compared with the non-administered group (Figure 9B, the second bar). No inhibiting effects were observed in application of the GABA_{A} receptor Cl- channel blocker (10 µM bicuculline or 100 µM picrotoxin) (Figure 9B, the fifth and sixth bars).

As shown in Figure 9B, the level of LDH released into the extracellular domain from the dead cells by NMDA exposure increased in about 4 fold compared with the control. Cell death was significantly inhibited by application of volume-sensitive C1-channel blocker (40 µM NPPB or 100 µM phloretin). There was no inhibition by application of the GABA_{A} receptor Cl- channel blocker (10 µM bicuculline or 100 µM picrotoxin). In the Figure, asterisk indicates a level of significant difference (p<0.05) from data on NMDA (before application of blocker).

These results suggest that application of volume-sensitive Cl- channel blocker during pathological hyperexcitation significantly inhibits necrotic neuronal cell death.

The embodiments and concrete examples of implementation discussed in the foregoing detailed explanation serve solely to illustrate the technical details of the present invention, which should not be narrowly interpreted within the limits of such embodiments and concrete examples, but rather may be applied in many variations within the spirit of the present invention, provided such variations do not exceed the scope of the patent claims set forth below.

### INDUSTRIAL APPLICABILITY

By inhibiting activity of volume-sensitive Cl- channel, the present invention can suppress (protection and/or relief against) excitotoxicity (neuronal injury in particular).

The invention does not target at receptors involved in neurotransmission, and therefore has no adverse effects on the normal operation of the nervous system. Further, the invention prevents brain edema and therefore suppresses further neuronal injury caused by increased intracranial pressure. The effects of the present invention are therefore multiple. This enables the present invention to be applied to clinical treatment relatively easily.

Further, the present invention is applicable to all forms of brain disorders in which neuronal hyperexcitation is observed, including, for example, seizure, stroke, and myocardial infarction, as well as brain ischemia or post-ischemic reperfusion that occurs during a heart transplant surgery or brain vessel anastomosis. Thus, by applying the present invention, the prognosis of patients suffering from these diseases can be greatly improved.

The present invention suppresses (protection and/or relief against) excitotoxicity without affecting normal operation of the nervous system. This enables the present invention to be applied to clinical treatment relatively easily. The invention is therefore very useful for the development of medicine intended to improve prognosis of patients suffering from all forms of brain disorders in which neuronal hyperexcitation is observed, including, for example, seizure, stroke, and myocardial infarction, as well as brain ischemia or post-ischemic reperfusion that occurs during a heart transplant surgery or brain vessel anastomosis.

## Claims

1. A therapeutic agent for vascular disease involving excitotoxicity, comprising a volume-sensitive Cl- channel blocker.

2. A therapeutic agent as set forth in claim 1, wherein the vascular disease comprises ischemic cerebrovascular disease.

3. A therapeutic agent as set forth in claim 1 or 2, wherein the cells comprise neurons.

4. A therapeutic agent as set forth in any one of claims 1 through 3, wherein the cell injury occurs during brain tissue swelling (brain edema), seizure, stroke, myocardial infarction, heart transplant, or brain vessel anastomosis.

5. A therapeutic agent as set forth in any one of claims 1 through 4, wherein the cell injury accompanies severe cell swelling.

6. A therapeutic agent as set forth in any one of claims 1 through 5, wherein the cell injury accompanies necrosis.

7. A therapeutic agent as set forth in any one of claims 1 through 6, wherein the cell injury accompanies a significantly increased level of propidium iodide (PI) permeability to plasma membrane.

8. A therapeutic agent as set forth in any one of claims 1 through 6, wherein the cell injury accompanies a significantly increased level of lactate dehydrogenase (LDH) release from cytoplasm.

9. A therapeutic agent as set forth in any one of claims 1 through 8, wherein the volume-sensitive Cl⁻ channel blocker comprises NPPB or phloretin.

10. A therapeutic method of a disease involving excitotoxicity, comprising a step of applying a volume-sensitive Cl⁻ channel blocker to cells.

11. A therapeutic method as set forth in claim 10, wherein the disease comprises ischemic cerebrovascular disease.

12. A therapeutic method as set forth in claim 10 or 11, wherein the cells comprise neurons.

13. A therapeutic method as set forth in any one of claims 10 through 12, wherein the cell injury occurs during brain tissue swelling (brain edema), seizure, stroke, myocardial infarction, heart transplant, or brain vessel anastomosis.

14. A therapeutic method as set forth in any one of claims 10 through 13, wherein the cell injury accompanies severe cell swelling.

15. A therapeutic method as set forth in any one of claims 10 through 14, wherein the cell injury accompanies necrosis.

16. A therapeutic method as set forth in any one of claims 10 through 15, wherein the cell injury accompanies a significantly increased level of propidium iodide (PI) permeability to plasma membrane.

17. A therapeutic method as set forth in any one of claims 10 through 15, wherein the cell injury accompanies a significantly increased level of lactate dehydrogenase (LDH) release from cytoplasm.

18. A therapeutic method as set forth in any one of claims 10 through 17, wherein the volume-sensitive Cl- channel blocker comprises NPPB or phloretin.

19. An evaluation method for evaluating cell death caused by hyperexcitation in an *in vitro* cell culture system, comprising:
a first measuring step of measuring cell death in cultured cells;
a stimulating step of subjecting the cells to excitotoxic stimulation;
a second measuring step of measuring cell death in the cells subjected to excitotoxic stimulation; and
a comparing step of comparing a first measurement value, obtained in the first measuring step, with a second measurement value, obtained in the second measuring step.

20. An evaluation method as set forth in claim 19, wherein the cells comprise neurons.

21. An evaluation method as set forth in claim 19 or 20, wherein the first measuring step and the second measuring step measure cell death using activity of mitochondrion dehydrogenase as a measure.

22. An evaluation method as set forth in any one of claims 19 through 21, wherein, in the first measuring step and the second measuring step, fragmentation of intranuclear DNA is used as a measure.

23. An evaluation method as set forth in any one of claims 19 through 21, wherein, in the first measuring step and the second measuring step, formation of varicosities in the cells is used as a measure.

24. An evaluation method as set forth in any one of claims 19 through 23, further comprising a step of detecting C1⁻current in varicosities.

25. An evaluation method as set forth in any one of claims 19 through 24, further comprising a step of applying a volume-sensitive Cl⁻ channel blocker to the cells after the first measuring step.

26. A screening method for screening a factor inhibiting cell injury caused by hyperexcitation in an in *vitro* cell culture system, comprising:
a first measuring step of measuring cell death in cultured cells;
a step of applying a candidate factor to the cultured cells;
a stimulating step of subjecting the candidate factor-applied cells to excitotoxic stimulation;
a second measuring step of measuring cell death in the cells subjected to excitotoxic stimulation; and
a comparing step of comparing a first measurement value, obtained in the first measuring step, with a second measurement value, obtained in the second measuring step.

27. A screening method as set forth in claim 25, wherein the cells comprise neurons.

28. A screening method as set forth in claim 26 or 27, further comprising a step of applying a volume-sensitive C1⁻channel blocker to the cells after the first measuring step.

29. An evaluation method for evaluating neuronal swelling caused by hyperexcitation in an *in vitro* cell culture system, comprising:
a stimulating step of subjecting cultured neurons to excitotoxic stimulation;
a step of visualizing dendrites of the neurons; and
a step of detecting formation of varicosities in the neurons.

30. An evaluation method as set forth in claim 29, wherein the visualizing step employs EGFP gene transfection.
